# EUROPEAN PATENT APPLICATION

(11) **EP 0 994 337 A2**
(43) Date of publication of application: **19.04.2000**
(21) Application number: 99307513.4
(22) Date of filing: 23.09.1999
(51) Int. Cl.: G01M 15/00

(54) **Electric chip detector**

(30) Priority: 09.10.1998 US 169427
(71) Applicant: GENERAL ELECTRIC COMPANY, Schenectady, NY 12345 (US)
(72) Inventor: Mahon, Wayne Errol, Lynnfield, Massachusetts 01940 (US); Bird, John Richard, Georgetown, Massachusetts 01833 (US)
(74) Representative: Pedder, James Cuthbert

(57) **Abstract**

In a chip detector circuit (10), a positive contact (12) and a negative contact (14) are separated by a chip gap (18). A magnet (22) is located between the positive and negative contacts (12, 14) for attracting metallic chips. Chips then collect at the magnet (22) until the chips bridge the chip gap (18), allowing electrical current to flow between the positive and negative contacts (12, 14). A resistor (26) is provided for allowing current flow between the positive and negative contacts (12, 14) without chip buildup, whereby when conductive chips collect on the chip detector (10) at the chip gap (18), apparent resistance drops below that of the resistor (26), and a signal (24) is provided to an indicator.

## Description

The present invention relates, in general to electrical chip detectors and more particularly to an improved electrical chip detector which allows for remote testability.

The invention herein described was made in the performance of work done under Navy Contract No. N00019-88-C-0050, awarded by the Department of the U.S. Navy under which the U.S. Government has certain rights.

In the normal process of manufacturing engines and in particular aircraft engines, it is customary to operate the engine on a test stand initially to make adjustments and ensure the proper installation of parts and accessories. During this operation, metal pieces or chips can accidentally find their way into the engine. If an excessive amount of this material appears, it can destroy the engine, unless detected early enough to shut down the engine.

In other situations, it is necessary to determine the presence of wear particles. During the normal sliding or rolling of one metal part against another, tiny metallic wear particles are produced in various sizes and shapes. Frequently, particularly in the case of machines such as engines, transmissions, and gear boxes, larger particles, termed "chips", are produced as the internal parts fatigue. When these chips are present, they indicate equipment damage and must be removed to prevent additional damage to the equipment.

An electrical chip detector is used to collect electrical conductive material in the form Of chips (fatigue flakes), such as bearing and gear material, and provide a warning of the component failure.

In would be desirable to have a chip detector capable of providing chip detection prior to loss of function of equipment. It would also be desirable to have such a chip detectpr which can provide chip detection early enough to avoid secondary damage if the signal is read and responded to in a timely manner. Finally, it would be desirable to have such a chip detector that is testable remotely.

The present invention provides for an electrical chip detector which can be used to collect electrical conductive bearing and gear material in the form of chips and also provide a warning of impending component failure. The chip detector of the present invention can be checked at engine start-up.

In accordance with one aspect of the present invention, a chip detector comprises a positive contact and a negative contact separated by a chip gap, with a magnet located between the positive and negative contacts for attracting metallic chips. Chips then collect at the magnet until the chips bridge the chip gap, allowing electrical current to flow between the positive and negative contacts. The chip detector further comprises a resistor for allowing current flow between the positive and negative contacts without chip buildup, whereby when conductive chips collect on the chip detector at the chip gap, apparent resistance drips below that of the resistor, and a signal is provided to an indicator.

The invention will now be described in greater detail, by way of example, with reference to the drawings, in which:-
Fig. 1 is a perspective view of an electrical chip detector constructed in accordance with the present invention; and
Fig. 2 is a schematic illustration of the chip detector circuit for operating the chip detector of Fig. 1.

The present invention will be described with respect to detection and collection of electrical conductive bearing and gear material, i.e., chips; those skilled in the art, however, will recognize that the principles of the present invention could be easily adapted or modified for use on a variety of components.

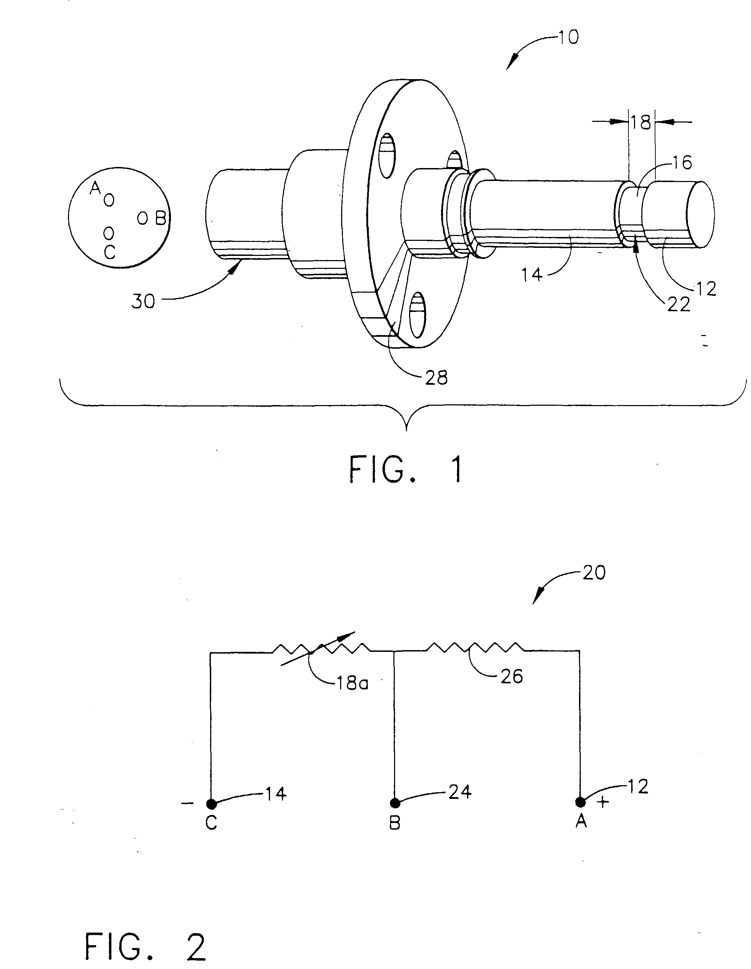

Referring to the drawings, Fig. 1 illustrates an electrical chip detector constructed in accordance with the present invention. The electrical chip detector 10 has two electrical contacts. Contact 12 is a positive contact and contact 14 is a negative contact. Contacts 12 and 14 are separated by insulators 15 and a minimum spacing or chip gap 18, resulting in an open electrical circuit, the electrical circuit 20 being schematically illustrated in Fig. 2.

Continuing with the drawings, located between the two contacts 12 and 14 is a magnet 22 used to attract metallic chips. The chips collect at the magnet 22 until they bridge the gap 18 between the two contacts 12 and 14. With the gap 18 bridged by electrically conductive chips, the electrical circuit 20 is closed at resistor 18a, indicative of gap 18, and electrical current is able to flow between the contacts 12 and 14. The closed circuit allows current flow to set-off a warning signal at signal means 24. This closed circuit can be sensed by current flow or reduced resistance across the chip detector.

In the prior art, because the circuit is normally open, the installation of the chip detector cannot be checked remotely and the chip detector is not testable. In accordance with the present invention, a testability feature is provided to allow the chip detector 10 to be checked at engine start-up.

A particularly novel concept of the present invention is the addition of an electrical resistor 26 to the chip detector circuit 20 to provide the engine start-up test capability. Resistor 26 creates a high resistance circuit which allows current flow through the contacts 12 and 14 in the normal installed condition, the normal installed condition being a "no chip" condition. In the prior art, resistor 26 being absent, no current flow is allowed through the contacts. The resistor 26, therefore, short circuits the gap 18. When conductive chips collect on the chip detector at chip gap 13, resistance of circuit 20 drops below that of the installed resistor 25. When the apparent resistance drops to a pre-specified trip setting, a signal or other warning is generated at a predetermined location, indicating an excess of electrical chips and impending component failure. The chip detector is powered by a known voltage which allows the trip setting to be pre-selected.

Also associated with the electrical chip detector 10 is a mounting flange 28 for locating and holding the chip detector in position, with the chip detector capable of being mounted to any component from which chips are to be collected. An electrical connector 30 provides a power source.

The chip detector 10 provides the advantage of remote testability. For example, a remotely located computer (remote to, for example, an aircraft engine or other internal component) can send an electrical pulse through the circuit 20. With the novel and useful inclusion of resistor 26, in accordance with the present invention, the signal can return, because the resistor short circuits the gap 18. The signal can indicate correct installation, as well as a "no chip" condition. Therefore, the electrical chip detector 10 according to the present invention provides the additional advantage of checking electrical cable installation without requiring the opening of engine bay doors, or intrusion into any other area being tested for chips.

While preferred embodiments of the present invention have been shown and described herein, it will be obvious to those skilled in the art that such embodiments are provided by way of example only. Numerous variations, changes, and substitutions will now occur to those skilled in the art without departing from the invention, and those skilled in the art will recognize that the principles of the present invention could be easily adapted or modified to achieve goals in various arrangements. For example, the chip detector can be used with a variety of components, including an engine oil system or other fluid system which would transmit magnetic electrical chips. Accordingly, it is intended that the invention be limited only by the scope of the appended claims.

## Claims

1. A chip detector (10) comprising:
a positive contact (12) and a negative contact (14) separated by a chip gap (18) ;
a magnet (22) located between the positive and negative contacts (12, 14) for attracting metallic chips, whereby the chips collect at the magnet (22) until the chips bridge the chip gap (18), allowing electrical current to flow between the positive and negative contacts (12, 14) ; and
a resistor (26) for allowing current flow between the positive and negative contacts (12, 14) without chip buildup, whereby when conductive chips collect on the chip detector (10) at the chip gap (18), apparent resistance drops below that of the resistor (26).

2. A chip detector (10) as claimed in claim 1 further comprising a signal means (24) for providing a warning signal when the apparent resistance drops to a specified limit, indicative of a trip setting.

3. A chip detector (10) as claimed in claim 1 Or 2 wherein the chip detector (10) is powered by a known voltage which allows the trip setting to be pre-selected.

4. A chip detector circuit (10) having a positive contact (12) and a negative contact (14) separated by a chip gap (18) and further having a magnet (22) located between the positive and negative contacts (12, 14) for attracting metallic chips, whereby the chips collect at the magnet (22) until the chips bridge the chip gap (18), allowing electrical current to flow between the positive and negative contacts (12, 14), the chip detector circuit (10) characterized by:
a resistor (26) for allowing current flow between the positive and negative contacts (12, 14) without chip buildup, whereby when conductive chips collect on the chip detector (10) at the chip gap (18), apparent resistance of the circuit (10) drops below that of the resistor (26); and
signal means (24) for providing an indication of resistance drop.

5. A chip detector circuit (10) as claimed in claim 4 wherein the resistor (26) provides an engine start-up test capability by closing the circuit.

6. A method for detecting chip build-up from an internal component of a machine, the method comprising the steps of:
defining a positive contact (12) and a negative contact (14);
separating the positive contact (12) from the negative contact (14) by a chip gap (18) ;
using a magnet (22) between the positive and negative contacts (12, 14) for attracting metallic chips, whereby the chips collect at the magnet (22) until the chips bridge the chip gap (18), allowing electrical current to flow between the positive and negative contacts (12, 14) ; and
providing a resistor (26) for allowing current flow between the positive and negative contacts (12, 14) without chip buildup, whereby when conductive chips collect on the chip detector (10) at the chip gap (18), apparent resistance drops below that of the resistor (26).

7. A method as claimed in claim 6 further comprising the step of providing a trip set warning signal (24) when the apparent resistance drops to a specified limit.

8. A method as claimed in claim 7 wherein the chip detector (10) is powered by a known voltage which allows the trip setting to be pre-selected.
